# EUROPEAN PATENT SPECIFICATION

(11) **EP 1 274 456 B1**
(45) Date of publication and mention of the grant of the patent: **29.12.2004**
(21) Application number: 01928654.1
(22) Date of filing: 19.04.2001
(51) Int. Cl.: A61K 45/00

(54) **USE OF RETINOID RECEPTOR ANTAGONISTS OR AGONISTS IN THE TREATMENT OF CARTILAGE AND BONE PATHOLOGIES**
DIE VERWENDUNG VON RETINOID ANTAGONISTEN ODER AGONISTEN FÜR DIE BEHANDLUNG VON GELENK-UND KNOCHENPATHOLOGIEN
UTILISATION D'ANTAGONISTES OU D'AGONISTES DU RECEPTEUR RETINOIDE DANS LE TRAITEMENT DE PATHOLOGIES DES CARTILAGES ET OS

(30) Priority: 20.04.2000 US 552823
(43) Date of publication of application: 15.01.2003
(73) Proprietor: Allergan, Inc., Irvine, California 92612 (US)
(72) Inventor: PACIFICI, Maurizio, Swarthmore, PA 19081 (US); CHANDRARATNA, Roshantha, A., Laguna Hills, CA 92653 (US)
(74) Representative: HOFFMANN - EITLE
(86) International application number: PCT/US2001/012742
(87) International publication number: WO 2001/080894

(56) References cited:
- WO-A-00/30635
- WO-A-00/53652
- CA-A- 2 254 429
- DE-A- 19 501 032
- US-A- 5 739 338
- US-A- 5 773 594
- US-A- 5 877 207
- US-A- 5 977 125

## Description

### CROSS REFERENCE TO RELATED APPLICATIONS

### Background of the Invention

Articular cartilage is a unique tissue present in the joints in the limbs, trunk and cervical region. The tissue is composed of articular chondrocytes and an abundant extracellular matrix that contains several well characterized macromolecules, including proteoglycan aggregates, hyaluronic acid, link protein and type II collagen fibrils. The chondrocytes are responsible for the synthesis, deposition and maintenance of the matrix components. The proteoglycan aggregates are large supramolecular structures that bind large quantities of water molecules and ions and provide the tissue with bioelasticity. The collagen fibrils form a three dimensional network that is able to withstand tensile and shear forces and provides the tissue with tensile strength. Together, the proteoglycan aggregates and collagen fibrils are responsible for a fundamental biomechanical property of articular cartilage, resilience. This property allows the tissue to undergo reversible changes in shape and volume that result from physical forces acting on the joints during movement, and thus permit normal functioning of the joints. Under normal healthy circumstances, articular chondrocytes remain active and phenotypically stable throughout life; in turn, this allows articular cartilage to maintain its structural and organization characteristics and to perform its biomechanical roles in the joints throughout life.

Endochondral ossification is the process by which the cartilaginous skeletal elements present in the embryo and growing organism are replaced by definitive bone elements. The process starts in the second half of embryogenesis and is concluded at the end of puberty when skeletal growth ceases. Endochondral ossification is a highly-regulated multistep process that involves several distinct steps of chondrocyte maturation and is best appreciable in long bone growth plates in the limbs. During endochondral ossification, resting immature chondrocytes first undergo a phase of rapid cell proliferation. The cells then withdraw from the cell cycle and enter a phase of active matrix production. Matrix components synthesized at this step are typical cartilage matrix macromolecules, including proteoglycans (aggrecan), type II collagen, link protein and hyaluronan. The postmitotic matrix-synthesizing cells then begin to enlarge in size and change from flat to oval-round in shape. This step is called the pre-hypertrophic stage and is characterized by synthesis of new proteins, including the signaling factor Indian hedgehog. The cells continue to enlarge and advance to their ultimate stage of maturation, the hypertrophic stage. The biosynthetic repertoire of hypertrophic chondrocytes changes dramatically, and the cells initiate production of various new proteins including: metalloproteases, type X collagen, alkaline phosphatase and annexin V-rich matrix vesicles. As they undergo these changes in biosynthesis, the hypertrophic chondrocytes also begin synthesis of bone-characteristic type I and III collagens and deposit apatite crystals in the matrix, thus transforming hypertrophic cartilage into a bone-like tissue. Finally, they undergo apoptosis. As a result, the tissue becomes amenable to invasion by bone and bone marrow precursor cells, which then proceed to remove the hypertrophic tissue and replace it with definitive bone tissue.

A large number of studies have been carried out during the last several years to identify and characterize the mechanisms regulating endochondral ossification. Interest in these mechanisms reflects the fact that defects in endochondral ossification are associated, and probably cause, congenital and acquired conditions of skeletogenesis (Jacenko et al., *J*. *Rheumatol*. 22:39-41 (1995)). Interestingly, several molecules have been shown to have a negative role in endochondral ossification and to limit the rates at which chondrocytes progress from the immature to the hypertrophic stage. These molecules include fibroblast growth factor-2 (FGF-2), fibroblast growth factor receptor-3 (FGF-R3), parathyroid-related protein (PTH-rP), and Indian hedgehog (IHH) (Coffin, et al., *Mol*. *Biol*. *Cell*, 6:1861-1873 (1995); Colvin et al., *Nature Genet*., 12:390-397 (1996); Vortkamp et al., *Science,* 273:613-622 (1996)). However, very few positive factors have been identified to date, which would have the critical role of counteracting the negative factors and allow the endochondral process to advance and reach its conclusion.

Pathologies associated with bone growth include osteoarthritis. Osteoarthritis is a degenerative disease of the joints that causes progressive loss of articular tissue. The disease, for which presently no cure or effective treatment exists, affects over 10% of the population over 60 years of age. Osteoarthritis is probably initiated by a number of factors, including mechanical insults derived from life-long use of the joints. Once articular cartilage is damaged, the disease progresses and numerous changes occur in the cells and matrix. At sites most affected by the disease, the articular chondrocytes can reinitiate proliferation and begin to acquire abnormal phenotypic traits. These include synthesis of type I and III collagens, cell hypertrophy, type X collagen synthesis, alkaline phosphatase activity increased proteolytic activity and even matrix mineralization (Hamennan, *New Engl*. *J. Med*. 320, 1322-1330 (1989); Nerlich, et al., *Vichows Archiv. B*. *Cell Pathol*. 63, 249-255 (1993); von der Mark, K. et al., *Acta Orthop*. *Scand.* 266, 125-129 (1995)). At the same time, while synthesis of proteoglycans increases, net proteoglycan content decreases because of increased matrix degradation by metalloproteases and other degradative enzymes. There are also reports that the articular chondrocytes can display signs of cellular degeneration and apoptosis. Once the articular cells disappear and the matrix degenerates, the tissue is replaced by non-functional scar tissue or even bony tissue.

Thus, a need exists for effective therapeutic methods for the treatment of bone pathologies.

DE 195 01 032 A discloses means for treating rheumatoid diseases, especially arthritis. It discloses a number of compounds, all of which are intended to render the matrixmetalloprotease collagenase-3 ineffective.

US5,977,125 discloses a number of compounds which are said to bind to retinoic acid receptors and considers their use in psoriasis and rheumatoid arthritis.

The present invention provides the use of claims 1 to 4. The retinoid receptor agonist is an RAR receptor agonist, and preferably an RAR alpha, beta, or gamma receptor agonist.

The present invention provides for enhancing bone growth comprising administering a therapeutically effective amount of a pharmaceutical composition comprising a retinoid receptor agonist.

In a further embodiment, the present invention provides for stimulating osteoprogenitor cells and osteoblasts by administering a therapeutically effective amount of a retinoid receptor agonist.

Bone growth related diseases include those involving pathological ossification such as osteoarthritis, multiple cartilaginous exostoses and osteoblastic tumors including osteoid osteoma, osteosarcoma and osteoma; and osteitis deformans (see generally, *Pathological Basis of Disease, Robbins, et al*. W.B. Saunders Co. (1979)). At the molecular level retinoids exert their biological effects through two families of nuclear receptors, retinoic acid receptors (RARs) and retinoid X receptors (RXRs), which belong to the superfainily of steroid/thyroid/vitamin D3 nuclear receptors.

RARs and RXRs are ligand-dependent transcription factors which' regulate gene expression in at least two different ways: (a) they upregulate the expression of genes by binding to the RA-responsive elements (RAREs) present in their promoters or (b) they down-regulate the expression of genes by antagonizing the enhancer action of certain other transcription factors, such as AP1. The distinct isotypes of RARs (α, β and γ) and RXRs (α, β and γ) are encoded by six separate genes. Each RAR isotype is further expressed as several isoforms differing in their N-terminal A region, which are generated by alternative splicing and/or by differential usage of more than one promotor. RARα is expressed as two main isoforms (α1 and α2). RARβ as four isoforms (β1, β2, β3 and β4) and RARγ as two main isoforms (γ1 and γ2). RARs are believed to function exclusively *in vivo* as RAR-RXR heterodimers.

As used herein, "agonist" means a compound that will stimulate the ligand-mediated transactivational activity of the specified retinoid receptor.

As used herein, "antagonist" means a compound that will inhibit or block the ligand-mediated transactivational activity of the specified retinoid receptor.

As used herein, "inverse agonist" means a compound that will decrease a basal level of transactivational activity of the specified retinoid receptor, wherein the basal level is that amount of transactivational activity observed in the absence of added agonist.

As used herein, the term "selective" means that a given ligand demonstrates at least about a 10 fold greater binding affinity, as indicated by, for example, K_{d} value, (dissociation constant) for one receptor subtype than for another receptor subtype.

As used herein, the term "specific" means that a given ligand demonstrates at least about a 500 fold greater binding affinity, and more preferably at least about a 1000 fold greater binding affinity, for one receptor subtype than for another receptor subtype.

As used herein, the term "treating" means reducing or slowing the progression of a disease. Alternatively, or additionally, the term means to remedy or cure a disease. Where the disease is tumor related, the term treating means to inhibit cancer cell growth and/or reduce the sign of a tumor.

As used herein, the term "bone healing" means a pathological condition where cartilage is converted into bone. Alternatively, or additionally, ther term means fracture repair.

As used herein, the term "non-union condition" means a pathological condition where the cartilage conversion to bone is inhibited or blocked.

As used herein, the term "osteoblasts" means the cells which continuously produce bone tissue in adults.

As used herein, the term "osteoclasts" means the cells which destroy bone.

The term "ameliorating" means reducing the symptoms associated with a particular disease, such as pain and inflammation.

Means for determining antagonist activity of a given agent or compound are known in the art. For example, a holoreceptor transactivation assay and a ligand binding 'assay which measure the antagonist/agonist like activity of the compounds of the invention, or their ability to bind to the several retinoid receptor subtypes, respectively, are described in published PCT Application No. WO 93/11755 (particularly on pages 30-33 and 37-41) published on Jun. 24, 1993, the specification of which is also incorporated herein by reference.

A pharmaceutically acceptable salt may be prepared for any compound in this invention having a functionality capable of forming a salt, for example, an acid functionality. A pharmaceutically acceptable salt is any salt which retains the activity of the parent compound and does not impart any deleterious or untoward effect on the subject to which it is administered and in the context in which it is administered.

Pharmaceutically acceptable salts may be derived from organic or inorganic bases. The salt may be a mono or polyvalent ion. Of particular interest are the inorganic ions, sodium, potassium, calcium, and magnesium. Organic salts may be made with amines, particularly ammonium salts such as mono-, di- and trialkyl amines or ethanol amines. Salts may also be formed with caffeine, tromethamine and similar molecules. Where there is a nitrogen sufficiently basic as to be capable of forming acid addition salts, such may be formed with any inorganic or organic acids or alkylating agent such as methyl iodide. In such cases, preferred salts are those formed with inorganic acids such as hydrochloric acid, sulfuric acid or phosphoric acid. Any of a number of simple organic acids such as mono-, di- or tri-acid may also be used.

Some of the compounds of the present invention may have trans and cis (E and Z) isomers. In addition, the compounds of the present invention may contain one or more chiral centers and therefore may exist in enantiomeric and diastereomeric forms. Still further oxime and related compounds of the present invention may exist in syn and anti isomeric forms. The scope of the present invention is intended to cover all such isomers per se, as well as mixtures of cis and trans isomers, mixtures of syn and anti isomers, mixtures of diastereomers and racemic mixtures of enantiomers (optical isomers) as well. In the present application when no specific mention is made of the configuration (cis, trans, syn or anti or R or S) of a compound (or of an asymmetric carbon) then a mixture of such isomers, or either one of the isomers is intended. In a similar vein, when in the chemical structural formulas of this application a straight line representing a valence bond is drawn to an asymmetric carbon, then isomers of both R and S configuration, as well as their mixtures are intended.

The present invention also provides pharmaceutical compositions comprising one or more compounds of the invention together with a pharmaceutically acceptable diluent or excipient. Preferably such compositions are in unit dosage forms such as tablets, pills, capsules (including sustained-release or delayed-release formulations), powders, granules, elixirs, tinctures, syrups and emulsions, sterile parenteral solutions or suspensions, aerosol or liquid sprays, drops, ampoules, auto-injector devices or suppositories; for oral, parenteral (e.g., intravenous, intramuscular or subcutaneous), intranasal, sublingual or rectal administration, or for administration by inhalation or insufflation, and may be formulated in an appropriate manner and in accordance with accepted practices such as those disclosed in *Remington's Pharmaceutical Sciences,* Gennaro, Ed., Mack Publishing Co., Easton PA, 1990. Alternatively, the compositions may be in sustained-release form suitable, for example, for once-weekly or once-monthly administration; for example, an insoluble salt of the active compound, such as the decanoate salt, may be adapted to provide a depot preparation for intramuscular injection. The present invention also contemplates providing suitable topical formulations for administration to, e.g. eye or skin or mucosa.

For instance, for oral administration in the form of a tablet or capsule, the active drug component can be combined with an oral, non-toxic pharmaceutically acceptable inert carrier such as ethanol, pharmaceutically acceptable oils, glycerol, water and the like. Moreover, when desired or necessary, suitable binders, lubricants, disintegrating agents, flavoring agents and coloring agents can also be incorporated into the mixture. Suitable binders include, without limitation, starch, gelatin, natural sugars such as glucose or beta-lactose, natural and synthetic gums such as acacia, tragacanth or sodium alginate, carboxymethylcellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

For preparing solid compositions such as tablets, the active ingredient may be mixed with a suitable pharmaceutical excipient, e.g., such as the ones described above, and other pharmaceutical diluents, e.g., water, to form a solid preformulation composition containing a homogeneous mixture of a compound of the present invention, or a pharmaceutically acceptable salt thereof. By the term "homogeneous" is meant that the active ingredient is dispersed evenly throughout the composition so that the composition may be readily subdivided into equally effective unit dosage forms such as tablets, pills and capsules. The solid preformulation composition may then be subdivided into unit dosage forms of the type described above containing from 0.1 to about 50 mg of the active ingredient of the present invention.

In another embodiment, the tablets or pills of the present composition may be coated or otherwise compounded to provide a dosage form affording the advantage of prolonged action. For example, the tablet or pill can comprise an inner core containing the active compound and an outer layer as a coating surrounding the core. The outer coating may be an enteric layer which serves to resist disintegration in the stomach and permits the inner core to pass intact into the duodenum or to be delayed in release. A variety of materials can be used for such enteric layers or coatings, such materials including a number of polymeric acids and mixtures of polymeric acids with conventional materials such as shellac, cetyl alcohol and cellulose acetate.

The liquid forms in which the present compositions may be incorporated for administration orally or by injection include aqueous solutions, suitably flavored syrups, aqueous or oil suspensions, and flavored emulsions with edible oils such as cottonseed oil, sesame oil, coconut oil or peanut oil, as well as elixirs and similar pharmaceutical carriers. Suitable dispersing or suspending agents for aqueous suspensions include synthetic and natural gums such as tragacanth, acacia, alginate, dextran, sodium carboxymethylcellulose, gelatin, methylcellulose or polyvinyl-pyrrolidone. Other dispersing agents which may be employed include glycerin and the like. For parenteral administration, sterile suspensions and solutions are desired. Isotonic preparations which generally contain suitable preservatives are employed when intravenous administration is desired. The compositions can also be formulated as an ophthalmic solution or suspension formation, i.e., eye drops, for ocular administration.

The term "subject," as used herein refers to an animal, preferably a mammal, most preferably a human, who has been the object of treatment, observation or experiment.

The term "therapeutically effective amount" as used herein means that amount of active compound or pharmaceutical agent that elicits the biological or medicinal response in a tissue, system, animal or human that is being sought by a researcher, veterinarian, medical doctor or other clinician, which includes alleviation of the symptoms of the disease being treated.

Advantageously, compounds of the present invention may be administered in a single daily dose, or the total daily dosage may be administered in divided doses two, three or four times daily. Furthermore, compounds for the present invention may be administered in intranasal form via topical use of suitable intranasal vehicles, or via transdermal routes, using those forms of transdermal skin patches well known to persons skilled in the art. To be administered in the form of a transdermal delivery system, the dosage administration will, of course, be continuous rather than intermittent throughout the dosage regimen, and dosage levels will require that this be taken into consideration when formulated.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound employed. A physician or veterinarian of ordinary skill can readily determine and prescribe the effective amount of the drug required to prevent, counter or arrest the progress of the disease or disorder which is being treated.

The instant invention relates to the use of retinoid receptor agonists as positive regulators of endochondral ossification, namely for (a)enhancing the reparative process during fracture repair, (b) treating congenital conditions in individuals who may exhibit poor or retarded growth and ossification, and (c) stimulating and modulating intramembrane ossification through treatment with retinoid receptor agonists. Congenital conditions of poor and retarded ossification may included, by way of example only and not of limitation, spondyloepiphyseal dysplasia congenita, skeletal dysplasias, hip dysplasia, and multiple epiphyseal dysplasias.

The synthesis and structures of exemplary retinoid receptor agonists is described, by way of example only and not of limitation, in U.S. Patent Nos. 5,808,124; 5,763,635; 5,747,542; 5,741,896; 5,723,666; 5,688,957; 5,618,943; 5,618,931; 5,616,712; 5,556,996; 5,543,534; 5,534,641; 5,514,825; 5,498,795; 5,498,755; 5,489,584; 5,475,022; 5,470,999; 5,451,605; 5,426,118; 5,399,561; 5,391,753; 5,346,915; 5,346,895; 5,344,959; 5,326,898; 5,134,159; 5,945,551; 5,015,658; 5,013,744; 5,006,550; 4,992,468; and 4,980,369, all of which are incorporated herein by reference in their entireties.

It is within the applicant's contemplation that any retinoid receptor agonist presently known in the art may be used in practicing the claimed methods.

All references cited are incorporated herein by reference in their entireties.

The invention is disclosed in further detail in the following examples, which are not in any way intended to limit the scope of the invention as claimed.

### Examples

### Example I - Bone Healing

A subject having a simple fracture of the humorus suffers from a congenital condition giving rise to poor natural bone healing. The patient's medical history shows that bone healing has typically taken 2-4 times longer in this patient than is seen in patients lacking such a condition.

Treatment is delayed for a period of time to permit infiltration of cartilage-forming cells into the space between the broken parts of the humorus. Bone healing is monitored daily by x-ray during the treatment regimen. Within 4 days of treatment normal matrix formation is seen, and the patient then treated by oral administration of a therapeutic amount of an RAR receptor agonist. The effective dosage is chosen in accordance with factors including the activity of the drug, and the age, weight, sex and medical condition of the patient and the oral route of administration. X-ray monitoring is continued during the course of therapy.

Monitoring during administration of the RAR agonist reveals that ossification occurs at a rate within the normal time course of bone healing for fractures of this type, particularly in comparison to the patient's prior medical history. The patient is able to resume use of the affected arm within a time considered normal.

## Claims

1. Use of an RAR receptor agonist or a pharmaceutically acceptable salt or ester thereof for the manufacture of a medicament for
(a) enhancing the reparative process during fracture repair,
(b) treating congenital conditions in individuals who exhibit poor or retarded growth and ossification,
(c) stimulating inframembrane ossification.

2. The use according to claim 1, wherein said RAR receptor agonist is an RARαβγ receptor agonist or a pharmaceutically acceptable salt or ester thereof.

3. The use according to any one of the preceding claims, wherein the pathology is a skeletal dysplasia.

4. Use according to any one of the preceding claims, wherein the medicament is for ossification by osteoprogenitor cells and osteoblasts.

## Patentansprüche

1. Verwendung eines RAR-Rezeptor-Agonisten oder eines pharmazeutisch annehmbaren Salzes oder Esters davon zur Herstellung'eines Medikaments zur
(a) Unterstützung des Heilungsvorgangs während der Bruchheilung,
(b) Behandlung angeborener Zustände von Individuen, die schlechte(s) oder verzögerte(s) Wachstum und Knochenbildung zeigen,
(c) Stimulierung der Intramembran-Knochenbildung.

2. Verwendung gemäss Anspruch 1, worin der RAR-Rezeptor-Agonist ein RARαβγ-Rezeptor-Agonist oder ein pharmazeutisch annehmbare(r/s) Salz oder Ester davon ist.

3. Verwendung gemäss mindestens einem der vorhergehenden Ansprüche, worin der pathologische Zustand eine Skelettdysplasie ist.

4. Verwendung gemäss mindestens einem der vorhergehenden Ansprüche, worin das Medikament zur Knochenbildung bei Osteoprogenitorzellen und Osteoblasten vorgesehen ist.

## Revendications

1. Utilisation d'un agoniste récepteur RAR ou de son sel ou ester pharmaceutiquement acceptable pour la fabrication d'un médicament destiné à
(a) activer le processus réparateur dans la cas de la réparation des fractures,
(b) traiter les conditions congénitales chez les individus qui présentent une croissance et ossification faible ou retardée,
(c) stimuler l'ossification intra - membranaire.

2. Utilisation selon la revendication 1, dans laquelle ledit agoniste récepteur RAR est un agoniste récepteur RARαβγ ou son sel ou ester pharmaceutiquement acceptable.

3. Utilisation selon l'une quelconque des revendications précédentes, dans laquelle la pathologie est une dysplasie du squelette.

4. Utilisation selon l'une quelconque des revendications précédentes, dans laquelle le médicament est destiné à l'ossification par les cellules ostéoprogénitrices et ostéoblastes.
